# EUROPEAN PATENT APPLICATION

(11) **EP 4 666 962 A1**
(43) Date of publication of application: **24.12.2025**
(21) Application number: 24183768.1
(22) Date of filing: 21.06.2024
(51) Int. Cl.: A61B 17/12, A61B 34/10, A61B 34/20, A61B 17/04, A61B 17/064, A61B 17/068

(54) **GAP COVERING EXPANDABLE IMPLANT FOR PREVENTING BLOOD LEAKAGE BETWEEN THERAPEUTIC DEVICE AND TISSUE AND ASSOCIATED DEVICES AND SYSTEMS**

(71) Applicant: Koninklijke Philips N.V., 5656 AG Eindhoven (NL)
(72) Inventor: TIKH, Mikhail Borisovich, Eindhoven (NL)
(74) Representative: Philips Intellectual Property & Standards

(57) **Abstract**

Devices, systems, and methods for preventing leakage through a therapeutic implant are provided. In an exemplary aspect, an apparatus is provided. The apparatus includes an implantable device configured to: contact heart tissue proximate to a left atrial appendage (LAA) with an occlusion device, cover a non-circular gap between the occlusion device and a wall of the LAA, and prevent blood flow into or out of the LAA. The implantable device includes a frame forming an umbrella-like shape having a bottom and sides curving upwards from the bottom, a mesh disposed over the bottom and at least a portion of the sides of the frame, and a fastener extending downward from the bottom of the frame and configured to attach to the occlusion device.

## Description

### TECHNICAL FIELD

The subject matter described herein relates to stopping leakage around therapeutic implants and, in particular, leakage around therapeutic cardiac implants such as occlusion devices. For example, devices can be catheter-deployed to stop such leaks by covering the gap between the therapeutic implant and the anatomy.

### BACKGROUND

Various types of therapeutic implants may be implanted into the heart to treat a variety of heart conditions. For example, for patients with diseases like atrial fibrillation, there may be a high risk of developing blood clots in the left atrial appendage (LAA). Thus, an occlusion device may be implanted at the opening of the LAA to prevent blood flow therein, thereby minimizing the risk of clots developing in the LAA.

However, when therapeutic implants such as these are implanted into a patient, there may be leakage between the exterior of the therapeutic implant and the wall of the anatomy of the heart. When a therapeutic implant is deployed in the heart, the circumference of the implant may not fully oppose the wall of the anatomy, resulting in one or more gaps between the implant and the wall. Undesirable blood flow may move through these gaps (i.e. leakage), resulting in lower performance of the implant. For example, in some cases, the therapeutic implant may not expand to the full size of the opening. This may occur because therapeutic implants are generally packaged and sold in discrete sizes, which are not tailored to the patient's specific anatomy. Thus, these gaps may form when an implant smaller than the size of the anatomy is deployed at the treatment site. In other cases, the therapeutic implant may have a circular shape and the anatomy has an ovular or non-circular shape. Thus, gaps may be created where the circumference of the circular-shaped plug and the non-circular shaped anatomy are not aligned. In either of these cases, the gaps between the therapeutic implant and the anatomy are generally non-circular and may be a variety of shapes, including crescent-shaped.

Thus, after deploying the therapeutic implant, it may be desirable (or necessary) to plug or fill these gaps to prevent leakage. Current methods of treating these gaps use circular devices or plugs. However, because the gaps are often non-circular, the circular plugs may not sufficiently plug the gap to prevent blood flow therethrough.

The information included in this Introduction section of the specification, including any references cited herein and any description or discussion thereof, is included for context and/or technical reference purposes only and is not to be regarded as subject matter by which the scope of the disclosure is to be bound or otherwise limited in any manner.

### SUMMARY

Aspects of the present disclosure are directed to an expandable implant, with associated systems, and a method to treat leakage between a therapeutic device and the anatomy using an expanding implantable device pre-loaded into a single delivery system. The device includes a catheter-based deployment device with an implantable device disposed therein. An actuator may be disposed within the catheter and may engage and/or contact the implantable device. The catheter can be placed over a gap between a therapeutic device and a wall of the heart (e.g. the LAA). The actuator and/or catheter can then be moved to push the implantable device out of the distal opening of the catheter so that the implantable device expands outward to contact the walls of the heart around the opening of the LAA to cover the therapeutic device and a gap between the LAA and the surrounding walls of the LAA. The actuator can then actuate a fastener of the implantable device to couple the implantable device to the therapeutic device. In this way, the implantable device is configured to cover the gap. Thus, the present disclosure advantageously provides an implantable device that can sufficiently prevent blood flow through one or more gaps of all shapes and sizes, including non-circular gaps.

In an exemplary aspect, an apparatus is provided. The apparatus includes an implantable device configured to: contact heart tissue proximate to a left atrial appendage (LAA) with an occlusion device, cover a non-circular gap between the occlusion device and a wall of the LAA, and prevent blood flow into or out of the LAA. The implantable device includes a frame forming an umbrella-like shape having a bottom and sides curving upwards from the bottom, a mesh disposed over the bottom and at least a portion of the sides of the frame, and a fastener extending downward from the bottom of the frame and configured to attach to the occlusion device.

In one aspect, the frame includes a plurality of wires. In one aspect, the frame is collapsable such that in a first configuration, the frame has a first diameter and, in a second configuration, the frame has a second diameter that is larger than the first diameter. In one aspect, the mesh is collapsable with the frame between the second configuration and the first configuration. In one aspect, the apparatus also includes a delivery catheter having a lumen configured to receive the implantable device before the implantable device contacts the tissue. In one aspect, the apparatus may also include an actuator positioned within the lumen and configured to provide distal motion to the implantable device to move the implantable device outside of the lumen. In one aspect, when the implantable device is positioned within the lumen, the frame is in the first configuration and, when the implantable device is moved outside the lumen, the frame is in the second configuration. In one aspect, the fastener is one of a suture or a hook. In one aspect, the mesh is disposed on a convex outer portion of the frame. In one aspect, the mesh is disposed over an entirety of the convex outer portion of the frame.

Aspects of the present disclosure are directed to a system for treating leakage between a therapeutic device and heart tissue of a patient. The system includes a catheter and an expandable implant. The catheter includes a tube defining a lumen and a distal opening at a distal end of the tube. The expandable implant is disposed in the lumen before the implant is implanted in the patient. The expandable implant includes a frame comprising a plurality of wires forming a bottom with sides curving upwards from the bottom such that the frame comprises a shape having a convex outer portion and a concave inner portion, a mesh disposed over at least a portion of the frame, and a fastener disposed at the bottom of the frame and configured to attach to the therapeutic device.

In one aspect, the mesh is disposed over the bottom and at least a portion of the sides of the frame. In one aspect, the frame is umbrella-shaped or bowl-shaped. In one aspect, the system also includes an actuator disposed within the lumen of the catheter and configured to move the implant distally within the lumen such that the implant exits the catheter via the distal opening. In one aspect, the implant includes a first, constrained configuration when the implant is disposed within the lumen of the catheter and a second, unconstrained configuration when the implant exits the distal end of the catheter. In one aspect, the implant is biased to expand from the first configuration to the second configuration. In one aspect, the plurality of wires of the frame are closer to each other in the first configuration than in the second configuration. In one aspect, the mesh is flexible such that it is moveable with the frame between the first configuration and the second configuration. In one aspect, the fastener includes a hook comprising at least one bendable wire. In one aspect, the therapeutic device includes an occlusion device and the occlusion device is positioned within a LAA of the patient.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of aspects of the present disclosure, e.g., as defined in the claims, is provided in the following written description of various examples and/or aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1A is a side view of a human heart according to aspects of the present disclosure.
Fig. 1B is a cross-sectional side view of a human heart according to aspects of the present disclosure.
Fig 2 is a cross-sectional side view of an occlusion device implanted in the left atrial appendage (LAA), according to aspects of the present disclosure.
Fig. 3A is a diagrammatic cross-sectional top view of the occlusion device along section line 7-7 in Fig. 6, according to aspects of the present disclosure.
according Fig. 3B is a diagrammatic cross-sectional top view of the LAA along section line 7-7 in Fig. 6, to aspects of the present disclosure.
Fig. 3C is a diagrammatic cross-sectional top view of the occlusion device deployed within the LAA, according to aspects of the present disclosure.
Fig. 4 is schematic, diagrammatic view of a system according to aspects of the present disclosure.
Fig. 5A is a front diagrammatic view of an exemplary implantable device, according to aspects of the present disclosure.
Fig. 5B is a side diagrammatic view of the exemplary implantable device of Figure 5A, according to aspects of the present disclosure.
Fig. 6A is a front diagrammatic view of another exemplary implantable device, according to aspects of the present disclosure.
Fig. 6B is a side diagrammatic view of the exemplary implantable device of Figure 6A, according to aspects of the present disclosure.
Fig. 7A is a cross-sectional diagrammatic view of an exemplary delivery catheter and actuator, according to aspects of the present disclosure.
Fig. 7B is a cross-sectional diagrammatic view of the exemplary delivery catheter and actuator shown in Fig. 7A with the implantable device, according to aspects of the present disclosure.
Fig. 8 is a flowchart of an exemplary method of deploying and implanting the implantable device, according to aspects of the present disclosure.
Figs 9A-9E are diagrammatic views of a series of steps in a method for delivering and deploying an implantable device using a delivery catheter and actuator, according to aspects of the present disclosure.
Fig. 10 is a diagrammatic cross-sectional side view of the LAA with an implantable device and therapeutic device implanted, according to aspects of the present disclosure.
Fig. 11 is a diagrammatic top view of the LAA with an implantable device and therapeutic device implanted, according to aspects of the present disclosure.
Fig. 12 is a schematic diagram of a processor circuit, according to aspects of the present disclosure.

### DETAILED DESCRIPTION

For the purposes of promoting an understanding of the principles of the present disclosure, reference will now be made to the examples illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one example and/or aspect may be combined with the features, components, and/or steps described with respect to other examples and/or aspects of the present disclosure. Additionally, while the description below may refer to blood vessels, it will be understood that the present disclosure is not limited to such applications. For example, the devices, systems, and methods described herein may be used in any body chamber or body lumen, including an esophagus, veins, arteries, intestines, ventricles, atria, or any other body lumen and/or chamber. For the sake of brevity, however, the numerous iterations of these combinations will not be described separately.

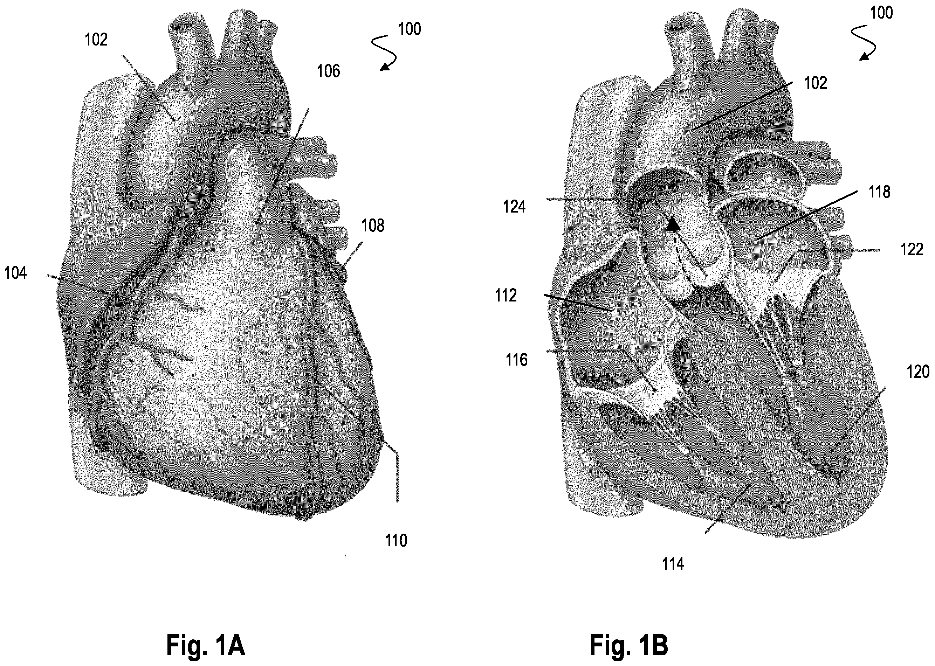

Fig. 1A is a side view of a human heart 100 according to aspects of the present disclosure. Visible are an aorta 102 from which stems a right coronary artery 104 and a left main coronary artery 106. The left main coronary artery 106 branches into a left circumflex coronary artery 108 and a left anterior descending coronary artery 110. The right coronary artery 104, the left main coronary artery 106, the left circumflex coronary artery 108, and a left anterior descending coronary artery 110 are the arteries that provide oxygen-rich blood to muscles of the human heart 100.

Fig. 1B is a cross-sectional side view of a human heart 100 according to aspects of the present disclosure. Visible are a right atrium 112 and a right ventricle 114. In that regard, oxygen-poor blood enters the human heart 100 in the right atrium 112 and travels to the right ventricle 114 through the tricuspid valve 116. The oxygen-poor blood leaves the right ventricle 114 and travels to the lungs. Also visible are a left atrium 118 and a left ventricle 120. In that regard, oxygen-rich blood is received from the lungs in the left atrium 118 and travels to the left ventricle 120 through the mitral valve 122. The oxygen-rich blood leaves the left ventricle 120 and goes out to the body through the aorta 102 via an aortic valve 124.

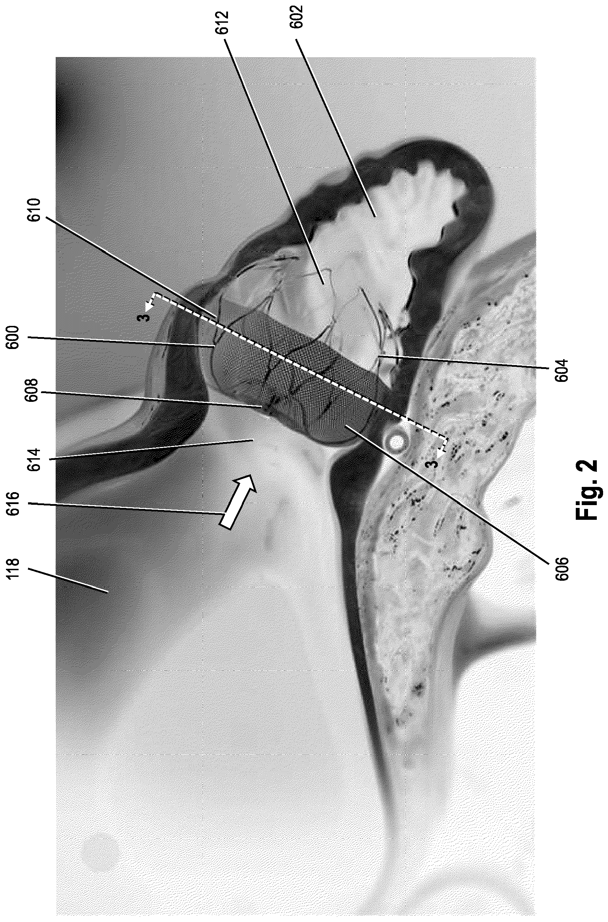

Fig. 2 is a cross-sectional view of an occlusion device 600 disposed in the left atrial appendage (LAA) of the left atrium 118 of the heart 100, according to aspects of the present disclosure. The occlusion device 600 may include a structure or cage 604 that forms an umbrella-like shape when expanded. The shape of the structure 604 may have a flat top 608 with rounded or curved sides 610 that curve around the bottom 612. The bottom 612 may be open. A mesh 606 may be disposed over and coupled to the top 608 and/or sides 610 of the structure 604. Thus, the mesh 606 may move with the structure 604 when it is expanded and/or contracted.

The occlusion device 600 may be delivered to the LAA 602 via a catheter such that the occlusion device 600 is folded within the catheter before deployment. When the distal end of the catheter is disposed proximate the opening 614 of the LAA 602, the occlusion device 600 may be deployed such that the top 608 is disposed proximate to or at the opening 614 and the bottom 612 and/or sides 610 are disposed within the LAA 602. When the occlusion device 600 exits the catheter, it may expand into the umbrella-like shape described above until the sides 610 contact the walls of the LAA 602. In some aspects, the top 608 and/or bottom 612 also contact the walls of the LAA 602. When the occlusion device 600 contacts the walls of the LAA 602, it may prevent blood flow into the LAA 602 (as shown by arrow 616), thereby minimizing the chance of blood clots developing within the LAA 602.

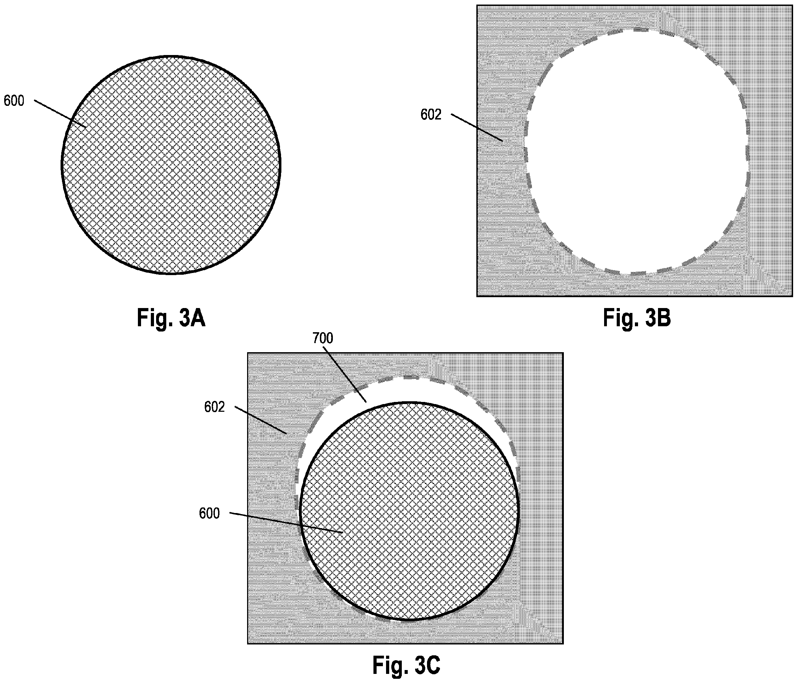

However, in some aspects, the occlusion device 600 may not completely close off the LAA 602. Figs 3A-3C illustrate an exemplary aspect in which the occlusion device 600 does not fully close off the LAA 602. Fig. 3A illustrates a diagrammatic cross-sectional view of the occlusion device 600 along the section line 3-3 in Fig. 2 from the direction indicated by the blood flow arrow 616, according to aspects of the present disclosure. This cross-section may be taken along the part of the occlusion device 600 with the largest diameter, which contacts the walls of the LAA. In the illustrated aspect, the cross-section of the occlusion device 600 is generally circular. In other aspects, the cross-section may be ovular, oblong, or any other suitable shape.

Fig. 3B illustrates a diagrammatic cross-sectional view of the walls of the LAA 602 along section line 3-3 in Fig. 2 from the direction indicated by the blood flow arrow 616, according to aspects of the present disclosure. In the illustrated aspect, the cross-section of the LAA 602 is oblong such that it is not perfectly circular. In other aspects, the cross-section may be circular, ovular, or any other suitable shape.

Fig. 3C illustrates a diagrammatic cross-sectional view of the occlusion device 600 deployed within the LAA 602 along section line 3-3 in Fig. 2 from the direction indicated by the blood flow arrow 616, according to aspects of the present disclosure. Because the cross-section of the occlusion device 600 is circular and the cross-section of the LAA 602 is non-circular, the occlusion device 600 does not continuously oppose the walls of the LAA 602. Thus, a gap 700 exists between part of the occlusion device 600 and the wall of the LAA 602. As shown in Fig. 3C, this gap 700 may be generally crescent-shaped or another non-circular shape. Blood flow may move through the gap between the device and the native tissue. Thus, when a gap 700 exists between the wall of the LAA 602 and the occlusion device 600, blood flow may enter and exit the LAA 602 via the gap 700. This may prevent the occlusion device 600 from fully sealing the LAA 602 as desired and may allow blood clots to form within the LAA 602.

Thus, when there are gaps (e.g. gaps 410, 420, 700) formed between the anatomy (e.g. heart wall 210 or LAA wall 602) and a therapeutic device (e.g. a replacement valve 300 or an occlusion device 600), this may prevent the therapeutic device from functioning as desired or needed. Thus, it may be advantageous to plug or close these gaps to prevent unwanted blood flow around the therapeutic device.

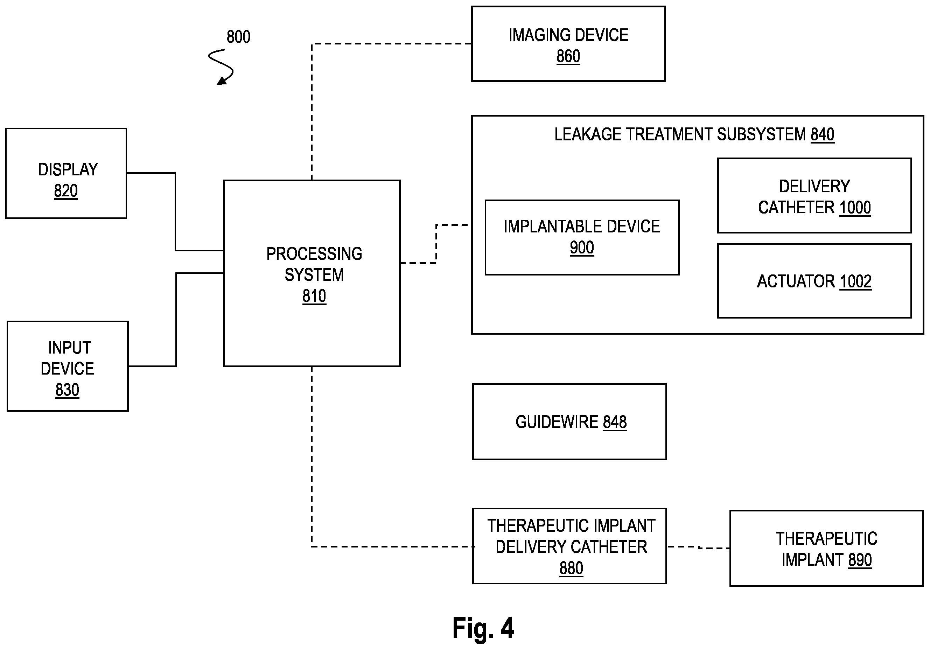

Fig. 4 is schematic, diagrammatic view of a system 800 that may prevent leakage between a therapeutic device or implant 890 and the anatomy of the patient, according to aspects of the present disclosure. The system 800 may be configured to evaluate (e.g., assess), display, and/or control (e.g., modify) one or more aspects of the delivery and/or deployment of a therapeutic implant 890 or the delivery and/or deployment of an implantable device 900. For instance, the system 800 may be utilized to monitor and/or control one or more portions of the delivery and/or deployment of a therapeutic implant 890 or the delivery and/or deployment of an expandable or implantable device 900. In this regard, the system 800 may be used to assess coronary vessels and/or heart tissue (e.g., the myocardium). As illustrated, the system 800 may include a processing system 810 in communication with a display device 820 (e.g., an electronic display or monitor), an input device 830 (e.g., a user input device, such as a keyboard, mouse, joystick, microphone, and/or other controller or input device), a leakage treatment subsystem 840 and/or an imaging device 860 (e.g., x-ray, computed tomography or CT, magnetic resonance imaging or MRI, etc.). As illustrated, the system 800 may further include a therapeutic implant delivery catheter 880 and a therapeutic implant 890. In some aspects, the therapeutic implant 890 may be an occlusion device 600 (e.g. for closing off the LAA 602). In other aspects, the therapeutic implant 890 may be a replacement valve (e.g. a prosthetic, mechanical, or donor valve). In some aspects, delivery and/or deployment of the therapeutic implant 890 via the therapeutic implant delivery catheter 880 may be completely mechanical (no connection to the processing system 810) or may be connected to processing system 810 (e.g., for control of movement and/or deployment of therapeutic implant). It is understood that other types of therapeutic implants and therapeutic implant delivery systems may be used instead of or in addition to those described herein.

The leakage treatment subsystem 840 includes a delivery catheter 1000, an actuator 1002, and an expanding/expandable implantable device 900 that can be deployed from the delivery catheter 1000 by being pushed out by the actuator 1002, as described in more detail below. In some aspects, delivery and/or deployment of the implantable device 900 via the delivery catheter 1000 may be completely mechanical (no connection to the processing system 810) or may be connected to processing system 810 (e.g., for control of movement and/or deployment of an implantable device 900).

Either or both of the delivery catheter 1000 and the therapeutic implant delivery catheter 880 may be guided over a guidewire 848.

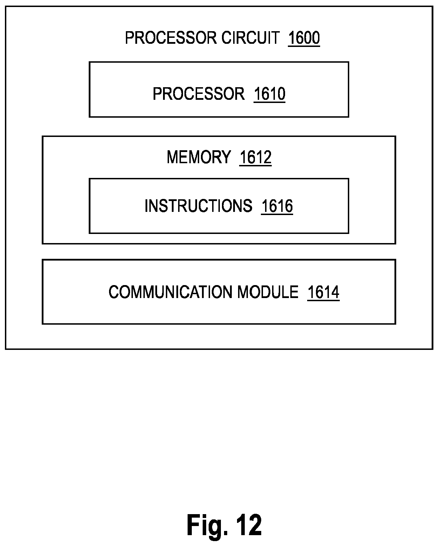

The processing system 810 is generally representative of any device suitable for performing the processing and analysis techniques disclosed herein. In some aspects, the processing system 810 includes a processor circuit, such as the processor circuit 1600 of Fig. 12, which is described in more detail below. In some aspects, the processing system 810 is programmed to execute steps associated with the data acquisition, analysis, and/or instrument (e.g., device) control described herein. Accordingly, it is understood that any steps related to data acquisition, data processing, instrument control, and/or other processing or control aspects of the present disclosure may be implemented by the processing system 810 (e.g., computing device) using corresponding instructions stored on or in a non-transitory computer readable medium accessible by the computing device. In some instances, the processing system 810 is a console device. Further, it is understood that in some instances the processing system 810 includes one or a plurality of computing devices, such as computers, with one or a plurality of processor circuits. In this regard, it is particularly understood that the different processing and/or control aspects of the present disclosure may be implemented separately or within predefined groupings using a plurality of computing devices. Any divisions and/or combinations of the processing and/or control aspects described below across multiple computing devices are within the scope of the present disclosure.

The system 800 is configured such that when the delivery catheter 1000 is positioned within the heart, images captured by the imaging system 860 can show the location and orientation of the delivery catheter 1000, and also potentially anatomical features such as the therapeutic implant 890, heart wall, and heart valve leaflets.

It is noted that block diagrams are provided herein for exemplary purposes; a person of ordinary skill in the art will recognize myriad variations that nonetheless fall within the scope of the present disclosure. For example, block diagrams may show a particular arrangement of components, subcomponents, modules, units, etc. It is understood that some aspects of the systems disclosed herein may include additional components, that some components shown may be absent from some aspects, and that the arrangement of components may be different than shown, while still performing the methods described herein.

It is understood that, in some instances, one or more components of the system 800 can operate without one or more other components of the system 800. For example, the leakage treatment subsystem 840, guidewire 848, therapeutic implant delivery catheter 880, and/or therapeutic implant 890 can be implemented without the processing system 810. For example, the leakage treatment subsystem 840, guidewire 848, therapeutic implant delivery catheter 880, and/or therapeutic implant 890 can be mechanical components that do not have signal communication with the processing system 810.

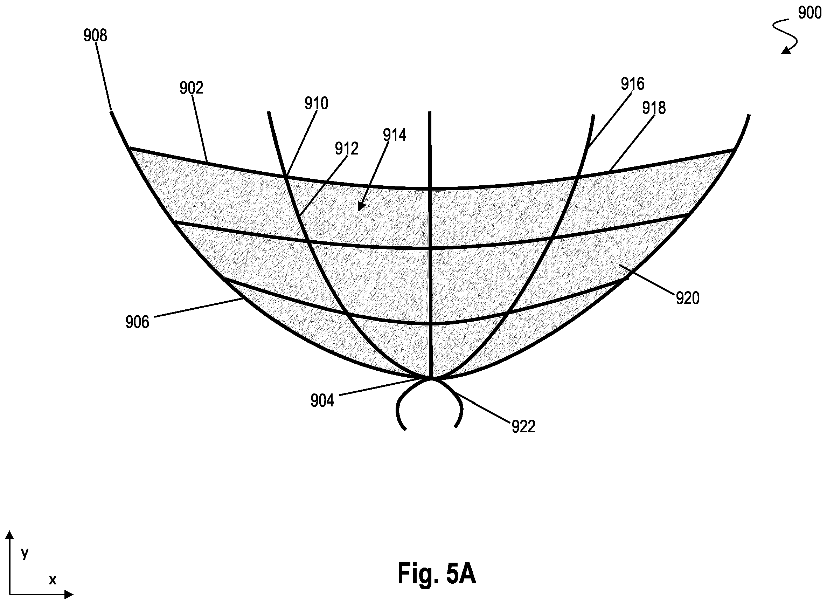

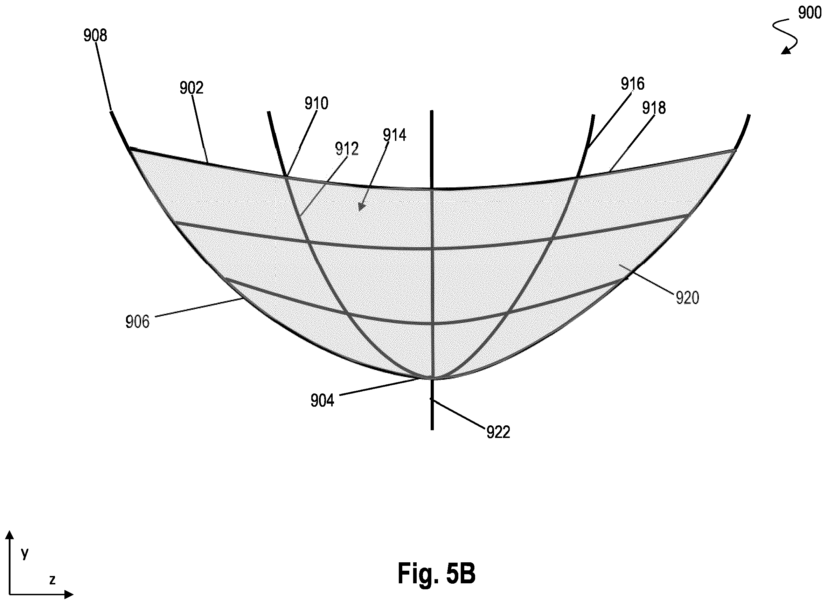

Figs 5A-5B illustrate a diagrammatic view of an implantable device 900 according to some aspects. Fig. 5A illustrates a front view of the implantable device 900. For example, the front view may illustrate the implantable device 900 along an x-y plane such that the x-dimension (i.e. lateral dimension) is oriented in the horizontal direction and the y-dimension (i.e. longitudinal dimension) is oriented in the vertical direction. Fig. 5B illustrates a side view of the implantable device 900. For example, the side view may illustrate the implantable device 900 along an y-z plane such that the z-dimension (i.e. depth or thickness dimension) is oriented in the horizontal direction and the y-dimension is oriented in the vertical direction.

The implantable device 900 may be formed of a single piece of wire 902 or a multiple pieces of wire 902 that are coupled together. The one or more wires may be bent, curved, or arranged into an umbrella-like or bowl-like shape. The umbrella-like or bowl-like shape may have a bottom 904 and sides 906 that extend upward from the bottom 904 such that a concave inner portion and a convex outer portion are formed. Thus, the top 908 of the shape may be open and the bottom 904 may be closed. The top 908 and bottom 904 may be spaced from each other along the y-dimension.

In some aspects, when multiple pieces of wire 902 are used, the wires 902 may be coupled by being bent or curved together or may be coupled using an adhesive or another coupling mechanism such as string, clips, staples, or any other suitable coupling mechanism. The wires 902 may be coupled such that they form a cage-like structure with coupling points 910 and struts 912 of wire extending therebetween that define open sections 914. This cage-like structure may have any suitable pattern. In the example illustrated in Figs 5A-5B, the cage-like structure includes a plurality of vertical wires 916 that curve upward from the bottom 904 along the sides 906 and a plurality of horizontal wires 918 that curve around the sides 906. The plurality of vertical wires 916 may contact each other at the bottom 904 and may be spaced in the x-dimension and the z-dimension as they extend upward from the bottom 904. The plurality of horizontal wires 918 may be spaced in the y-dimension and may not contact each other. In some aspects, a horizontal wire 918 may not be disposed at the top 908 of the cage-like shape such that the plurality of vertical wires 916 extend past the top-most horizontal wire 918 to the top 908, as illustrated in Figs 5A-5B. In other aspects, a horizontal wire 918 may be disposed at the top 908 of the cage-like structure such that a rim is formed around the open top 908. This may form a pattern that looks like a web with four-sided or three-sided open sections 914. This pattern may be formed in any suitable way. For example, in some aspects, each strut 912 may be a separate wire. In some aspects, two opposing vertical wires 916 may be formed of a single wire.

Mesh 920 (illustrated in light gray herein) may be coupled to the cage-like structure formed by the wires 902. The mesh 920 may cover the bottom 904 and at least part of the sides 906 of the cage-like structure. In the illustrated example, the mesh 920 only covers a portion (approximately three-quarters) of the sides 906. However, in other aspects, the mesh 920 may cover the entirety of the sides 906. In some aspects, the mesh 920 may be disposed on the exterior of the cage-like structure such that it covers at least a portion of the convex outer portion. In other aspects, the mesh 920 may be disposed on the interior of the cage-like structure such that it covers at least a portion of the concave inner portion. In some aspects, a single piece of mesh 920 may be used. In other aspects, multiple pieces of mesh 920 may be used. In some aspects, one piece of mesh 920 may cover at least a portion of the convex outer portion and another piece of mesh may cover at least a portion of the concave inner portion.

In some aspects, the mesh 920 may be configured to block blood flow through the implantable device 900. In some aspects, the mesh may 920 encourage endothelialization around the implantable device 900, which may further prevent blood flow through the implantable device 900. In some aspects, the mesh 920 may be at least partially permeable. In some aspects, the mesh 920 may be a fabric.

The wires 902 may be formed into the cage-like structure such that the structure is flexible. In some aspects, the sides 906 of the structure may be moved and/or flexed inwards such that the plurality of vertical wires 916 and/or the plurality of horizontal wires 918 are moved closer together (e.g. a distance between the wires 916, 918 is decreased) and/or the size of the open sections 914 decreases. During flexion, the position of the coupling points 910 and struts 912 may change, move, slide, or bend relative to each other. In some aspects, when the structure is flexed, the wires 902 may be formed such that they are biased outward to the original shape, as described in more detail below.

Mesh 920 may also be flexible such that, as the cage-like structure is moved or flexed, the mesh 920 is moved or flexed with the wires 902. Thus, when the sides 906 of the structure are moved inwards, the mesh 920 may be moved, flexed, and/or folded inwards with the wires 902. In some aspects, the mesh 920 may also be biased to return to its original shape.

The implantable device 900 may include an attachment structure, coupler, or fastener 922 configured to couple the implantable device 900 to a therapeutic implant 890 implanted into a patient. The fastener 922 may extend downward from the bottom 904 of the implantable device 900. In some aspects, the fastener 922 may be disposed on the center of the bottom 904. In some aspects, the fastener 922 may be disposed on any part of the bottom 904.

The fastener 922 may include any suitable structure capable of coupling the implantable device 900 to the therapeutic implant 890. For example, as illustrated in Figs 5A-5B, the fastener 922 may include one or more hooks. As illustrated, the fastener 922 includes two hooks. The hooks may be oriented towards each other such that there is an opening therebetween. The hooks may be configured to bend towards each other to close the opening around a portion of the therapeutic implant 890 to couple or attach the implantable device 900 to the therapeutic device 890.

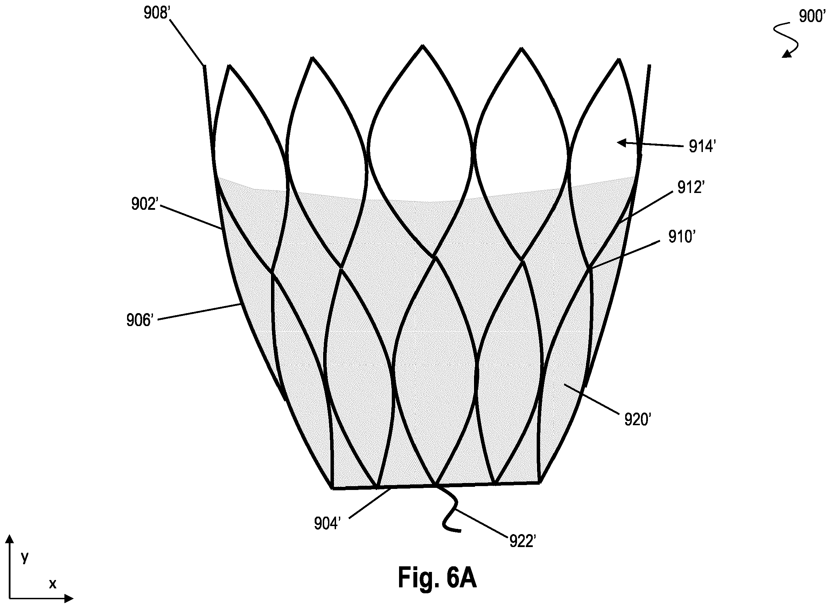

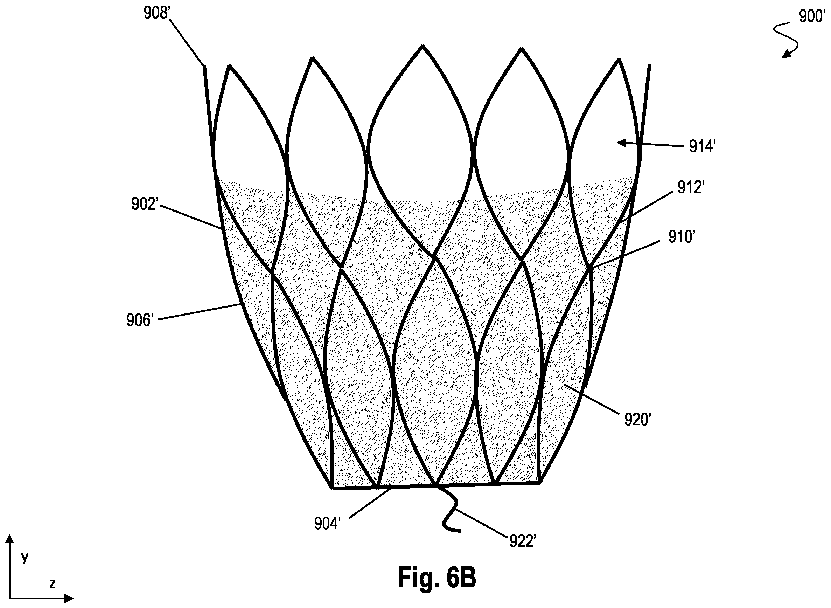

Figs 6A-6B illustrate a diagrammatic view of another example of an implantable device 900' according to some aspects. Fig. 6A illustrates a front view of the implantable device 900'. For example, the front view may illustrate the implantable device 900' along an x-y plane such that the x-dimension (i.e. lateral dimension) is oriented in the horizontal direction and the y-dimension (i.e. longitudinal dimension) is oriented in the vertical direction. Fig. 6B illustrates a side view of the implantable device 900'. For example, the side view may illustrate the implantable device 900' along an y-z plane such that the z-dimension (i.e. depth or thickness dimension) is oriented in the horizontal direction and the y-dimension is oriented in the vertical direction.

The implantable device 900' illustrated in Figs 6A-6B may be similar to the implantable device 900 illustrated in Figs 5A-5B but with a different pattern for the cage-like structure. For example, the one or more wires 902' of the implantable device 900' illustrated in Figs 6A-6B may be bent and/or coupled together such that they form a diamond-like pattern. The diamond-like pattern may be formed of a plurality of diamond-like shapes. Each diamond-like shape may include between 1-4 coupling points 910' and 1-4 struts 912' extending therebetween, defining a diamond-shaped open section 914'. Each diamond-like shape may be coupled to one or more other diamond-like shapes to form the diamond-like pattern. In some aspects, each diamond-like shape is formed of a single wire 902'. In other aspects, each diamond-like shape is formed of multiple wires 902' such as, for example, 2, 3, or 4 wires 902'. In some aspects, one or more wires 902' may be used to form multiple diamond-like shapes. For example, two wires 902' may be used together to form two diamond-like shapes, with one wire 902' forming a first side of each diamond-like shape and a second wire 902' forming a second side of each diamond-like shape.

As in the implantable device 900 illustrated in Figs 5A-5B, the implantable device 900' illustrated in Figs 6A-6B may include a mesh 920' that is disposed over the bottom 904' and at least a portion of the sides 908' of the cage-like structure, as described in more detail above.

As in the implantable device 900 illustrated in Figs 5A-5B, the implantable device 900' illustrated in Figs 6A-6B may be flexible such that the sides 906' may be moved and/or flexed inwards. Similarly, when the structure is flexed, the wires 902' may be formed such that they are biased outward to the original shape.

The implantable device 900' illustrated in Figs 6A-6B may have a bottom 904' that is flatter (i.e. has a lower curvature) than the bottom 904 of the implantable device 900 illustrated in Figs 5A-5B. The sides 906' of the implantable device 900' shown in Figs 6A-6B may extend upward from the relatively flat bottom 904' to a top 908' of the implantable device 900'. In some aspects, the sides 906' of the implantable device 900' illustrated in Figs 6A-6B may be flatter (i.e. have a lower curvature) than the sides 906 of the implantable device 900 illustrated in Figs 5A-5B. In some aspects, a rim (not shown) may be disposed around the top 908' of the implantable device 900'.

The implantable device 900' illustrated in Figs 6A-6B may include a fastener 922' that is a suture. The suture may be a flexible thread-like material that can be sewn or sutured to the therapeutic implant 890. In some aspects, the suture may be coupled to the bottom 904' of the implantable device 900' before it is implanted into the patient's body. In other aspects, the suture is coupled to the implantable device 900' and the therapeutic implant 890 after the implantable device 900' is implanted into the body proximate the previously implanted therapeutic device 890.

However, any suitable fastener 922 may be used to couple an implantable device 900 described herein to the therapeutic implant 890. In some aspects, the fastener 922 may be staples, an adhesive, a threaded connection (with a first female/male threading on the implantable device 900 that threadedly couples to a second male/female threading on the therapeutic implant 890), interlocking locking structures, or any other structure. In some embodiments, the implantable device 900 may have more than one fastener 922.

The wires 902 may be formed of any suitable material. For example, the wire may be formed of a metal or metal alloy such as stainless steel, nickel, titanium, nitinol, cobalt, chromium, any alloy thereof, or any other suitable metal/metal alloy. In other aspects, the wires 902 may be formed of a polymer such as ultra-high molecular weight polyethylene, polyether ether ketone, shape memory polymers, or any other suitable polymer. Mesh 920 may be formed of any suitable material. For example, mesh 920 may be formed of polyester, propylene, polyglycolic acid, animal-derived materials (e.g. porcine or bovine), or any other suitable material. The fastener 922 may be formed of any suitable material. For example, the fastener 922 may be formed of wire and may be the same material as the wires 902 of the structure. In some aspects, the fastener 922 may be formed of an absorbable material such as an absorbable sutures including, for example, polydioxanone, polyglycolic acid, polyglyconate, polylactic acid, collagen (which may be derived from an animal), or any other suitable material.

In some aspects, the at least part of the wires 902 may include barbs, fibers, or other structures that configured to improve engagement or attachment to the therapeutic implant 890 and/or tissue by preventing movement of the implantable device 900 once it is implanted. In some aspects, the barbs, fibers, or other structures may increase endothelialization around the implantable device 900. The barbs, fibers, or other structures may be formed of any suitable material including for example, any of the metal, metal alloys, or polymers listed above or an absorbable material such as an absorbable sutures including, for example, polydioxanone, polyglycolic acid, polyglyconate, polylactic acid, collagen (which may be derived from an animal), or any other suitable material.

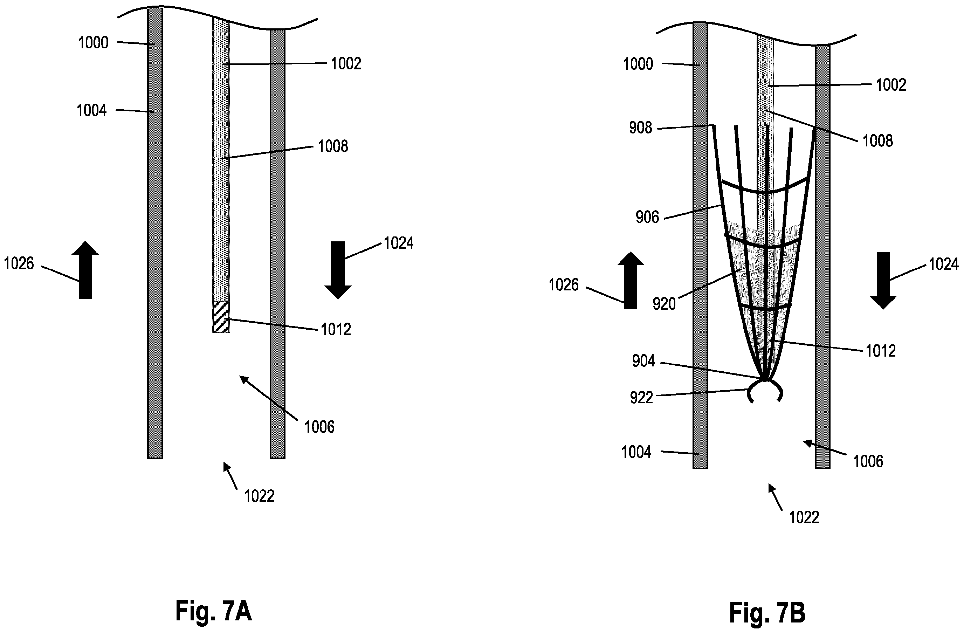

The implantable device 900 may be delivered to the site of the therapeutic implant 890 and deployed using a delivery catheter 1000 and a push rod or actuator 1002. Figs 7A-7B illustrate a delivery catheter 1000 and an actuator 1002 according to some aspects. Fig. 7A illustrates a cross-sectional view of the delivery catheter 1000 and actuator 1002 without the implantable device 900 and Fig. 7B illustrates the delivery catheter 1000 and actuator 1002 with the implantable device 900. In Figs 7A-7B, the illustrated implantable device 900 is similar to the implantable device 900 illustrated in Figs 5A-5B. However, it should be understood that the catheter 1000 and actuator 1002 may be used similarly for any implantable device described herein including the implantable device 900' illustrated in Figs 6A-6B. The delivery catheter 1000 may include a tube 1004 having a lumen 1006 extending from a proximal end to a distal end of the tube 1004. The distal end comprises a distal opening 1022 through which the implantable device 900 is pushed or ejected during deployment. The actuator 1002 may be disposed within the lumen 1006 of the tube 1004.

In some aspects, the actuator 1002 may be configured to move and/or push the implantable device 900 when the implantable device 900 is disposed within the lumen 1006 of the catheter 1000. For example, the actuator 1002 may be configured to push the implantable device 900 out of the catheter 1000 and implant the implantable device 900 into the therapeutic device 890 and/or tissue. Thus, the actuator 1002 may be configured to move distally (as shown by arrow 1024) and/or proximally (as shown by arrow 1026). In some aspects, the actuator 1002 may be configured to hold the implantable device 900 within the catheter 1000 as the catheter 1000 is moved to the site of the therapeutic implant 890. In some aspects, the actuator 1002 is configured to hold the implantable device 900 relatively still or steady while the tube 1004 of the catheter 1000 is moved to position the implantable device 900 outside of the lumen 1006. Thus, the catheter 1000 may be configured to move distally (as shown by arrow 1024) and/or proximally (as shown by arrow 1026). In some aspects, both the actuator 1002 and the catheter 1000 are moveable.

As shown in Fig. 7B, the implantable device 900 may be disposed within the lumen 1006 of the catheter 1000 such that the top 908 points to towards the proximal end of the catheter 1000 and the bottom 904 points to the distal end of the catheter 1000. When the implantable device is inserted into the lumen 1006 of the catheter 1000, the walls of the catheter 1000 may apply a force to the sides 906 of the implantable device 900 to move or flex the sides inward such that the implantable device is elastically deformed into a first, constrained configuration. Thus, the implantable device 900 may be constrained or flexed such that it is spring-loaded within the lumen 1006 of the catheter 1000. In the first configuration, the implantable device 900 may have a diameter that is smaller than the diameter of the original shape. For example, the diameter of the implantable device 900 in the first configuration may be equal to or smaller than a diameter of the lumen 1006.

In some aspects, a tip 1012 of the actuator 1002 may contact the implantable device 900. In some aspects, the tip 1012 may contact the inner concave portion of the implantable device 900 opposite the bottom 904. In some aspects, the tip 1012 may contact the inner concave portion of the implantable device 900 at the sides 906. In some aspects, the tip 1012 may contact the top 908 of the implantable device 900.

When the actuator 1002 pushes the implantable device 900 out of the distal opening 1022 of the catheter 1000, the walls of the catheter 1000 will no longer constrain the structure of the implantable device 900. Thus, because the implantable device 900 is biased to return to its original shape, the implantable device 900 may move to a second, unconstrained configuration where it has its original shape.

In some aspects, the tip 1012 of the actuator 1002 may be configured to actuate the fastener 922 to couple the implantable device 900 to the therapeutic implant 890. In some aspects, when the fastener 922 includes one or more hooks as shown in Figs 5A-5B, the tip 1012 may be configured to bend, move, or flex, the hooks towards each other. In some aspects, when the fastener 922 includes a suture as shown in Figs 6A-6B, the tip 1012 may be configured to thread the suture through the therapeutic implant. In other aspects, the actuator 1002 may be moved or removed so that a separate device may be used to actuator the fastener 922.

The tip 1012 may have any suitable structure. For example, the tip 1012 may simply include a distal surface that contacts and pushes against the implantable device 900. In other examples, the tip 1012 may include a hook that hooks onto the wires 902 and/or mesh 920 of the implantable device 900 to engage and/or hold the implantable device 900. In some aspects, the tip 1012 may include a needle and/or needle holder that contacts the implantable device 900 to push it out of the catheter 1000 and engages a suture fastener 922 to couple the suture (and thereby the implantable device 900) to the occlusion device 600. In some aspects, the tip 1012 may include a forceps that include two arms that can be moved towards each other to grab the implantable device 900 to move it out of the catheter 1000 and may actuate the fastener 922 (e.g. hooks or suture) to couple the fastener 922 (and thereby the implantable device) to the occlusion device 600.

The actuator 1002 may have any suitable shape or form. In some aspects, the actuator 1002 comprises an elongate member 1008 such as a wire, tube, or any other suitable elongate member. The elongate member 1008 may include a stiffness and/or rigidity such that the actuator 1002 can hold and/or move the implantable device 900.

In some aspects, the delivery catheter 1000 and/or elongate member 1008 of the actuator 1002 may be flexible elongate members that are sufficiently flexible to move through bends in a patient's vasculature (e.g. tortuous vasculature). Thus, the delivery catheter 1000 may be steerable through the vasculature of a patient. In some aspects, the catheter 1000 and/or elongate member 1008 may be formed of any suitable flexible material. For example, the catheter 1000 and/or elongate member 1008 may be formed of a polymer, including, for example, one or more of silicone, polyurethane (PE), polyethylene (PE), polyvinylchloride (PVC), PTFE, nylon, polyether block amide (e.g., Pebax^{®}), etc. In some aspects, the catheter 1000 and/or elongate member 1008 may be formed of a metal or metal alloy, including, for example, stainless steel, nickel, titanium, nitinol, cobalt, chromium, any alloy thereof, or any other suitable metal/metal alloy. In some aspects, the catheter 1000 and/or elongate member 1008 may include combination of a polymer with a metal or metal alloy. In some aspects, the catheter 1000 and elongate member 1008 are formed of the same material. In other aspects, the catheter 1000 and elongate member 1008 are formed of different materials.

The delivery catheter 1000 and the actuator 1002 may be used to deliver the implantable device 900 to the location of the gap 700 between the therapeutic device 890 and the surrounding tissue. The gap 700 may be between an occlusion device 600 and the opening or mouth of the LAA 602, as shown in Figs 3A-3B. However, it should be understood that the implantable device 900 may be used for any suitable therapeutic device 890 where it is desirable to cover a gap or leakage between the therapeutic device 890 and the surrounding tissue.

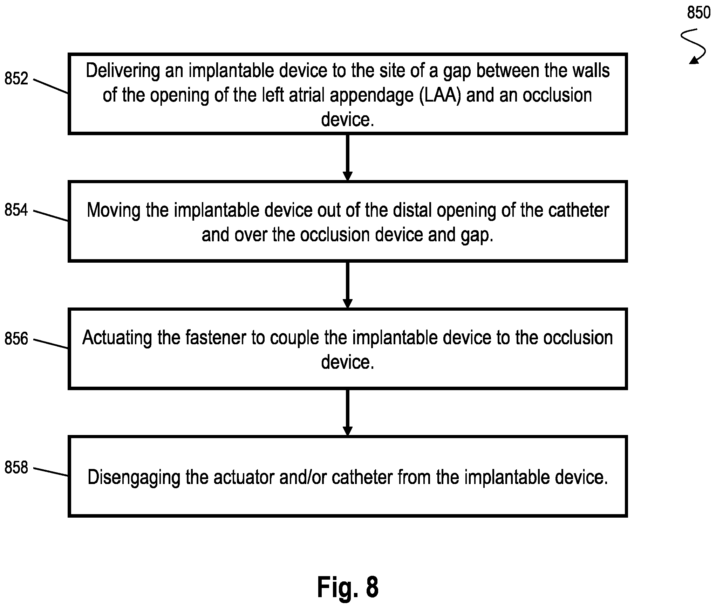

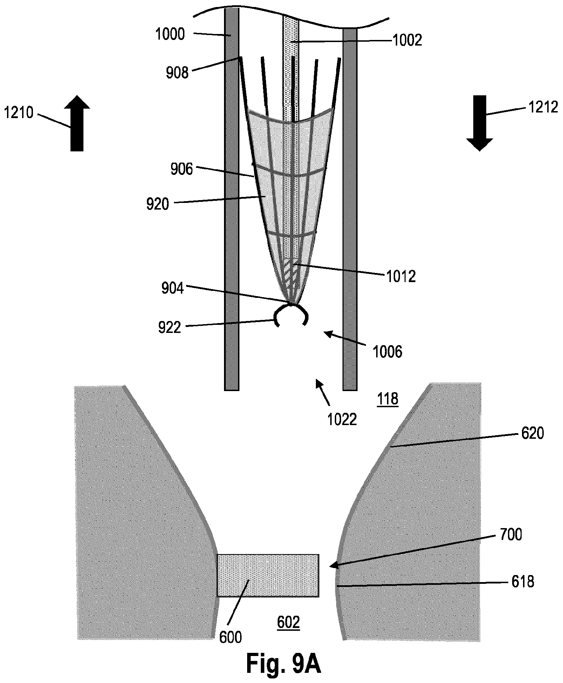

Fig. 8 is a flow chart illustrating an example method 850 of deploying and implanting the implantable device 900 to cover a gap 700 between an occlusion device 600 and the walls 618 around the opening 614 of the LAA 602, according to some aspects. The method 850 will be described in reference to Figs 9A-9E, which illustrate various steps in the method 850. Any suitable implantable device 900 described herein may be implanted according to the illustrated method, including those described in reference to Figs 5A-6B. Any suitable delivery catheter 1000 described herein may be used to deliver and/or deploy the implantable device 900 according to the illustrated method, including those described in reference to Figs 7A-7B. Any suitable actuator 1002 described herein may be used to deliver and/or deploy the implantable device 900 according to the illustrated method, including those described in reference to Figs 7A-7B. Although Figs 8-9E are described in reference to an occlusion device 600 implanted into the LAA 602, it should be understood that the method described may be performed to cover a gap between any therapeutic device 890 and the cardiac tissue.

Step 852 of the method 850 may include delivering the implantable device 900 to the site of a gap 700 between an occlusion device 600 and the walls 618 of the opening 614 of the LAA 602. Fig. 9A illustrates a diagrammatic view of the implantable device 900 being delivered to the gap 700 between an occlusion device 600 and the walls 618 of the opening 614 of the LAA 602. A first cardiac volume (e.g. the LAA 602) may be disposed on a first side of the occlusion device 600 and a second cardiac volume (e.g. the left atrium 118) may be disposed on a second side of the occlusion device 600. The occlusion device 600 may be intended to prevent blood flow from the left atrium 118 into the LAA 602.

In some aspects, the occlusion device 600 may have been previously implanted into the patient during a separate procedure. For example, the occlusion device 600 may have been implanted between one day and six months before the current method is performed (i.e. the procedure to prevent leakage through the gap 700). In these aspects, a physician may notice (e.g. during a follow up appointment or check-up) that the occlusion device 600 has not been performing properly and/or has a leak. Thus, the physician may decide to perform the current method to treat and/or minimize/eliminate the effect of the gap 700 to improve functioning of the occlusion device 600. In some aspects, any degree of endothelialization around the occlusion device 600 before the current method is performed such that one or more endothelial cells are disposed on the occlusion device 600 and/or walls 618 of the LAA 602.

In other aspects, the occlusion device 600 may be implanted during the same procedure as the current method is performed. In these aspects, a physician may notice a leak or gap 700 between the occlusion device 600 and the walls 618 of the LAA 602 after the occlusion device 600 has been deployed. Thus, the physician may implement the current methods before the procedure is completed to treat and/or minimize/eliminate the effect of the gap 700 so that the occlusion device 600 will perform properly.

As shown in Fig. 9A, the catheter 1000 may be moved proximate to the gap 700 such that the distal opening 1022 is oriented towards the gap 700. The catheter 1000 may be disposed within the left atrium 118 and may face the opening 614 of the LAA 602. The actuator 1002 may be disposed within the catheter 1000 such that the tip 1012 contacts, engages, and/or holds the implantable device 900, as shown in Fig. 7B. The implantable device 900 may be disposed in the catheter 1000 such that the top 908 is pointed proximally (as shown by proximal arrow 1210) relative to the catheter 1000 and the bottom 904 is pointed distally (as shown by distal arrow 1212) towards the distal opening 1022 of the catheter 1000, as shown in Fig. 7B. As described above, the walls of the catheter 1000 may apply a force to the sides 906 and/or top 908 of the implantable device 900 to hold the implantable device in a first, constrained configuration. Thus, the implantable device 900 may be constrained or flexed such that it is spring-loaded within the lumen 1006 of the catheter 1000.

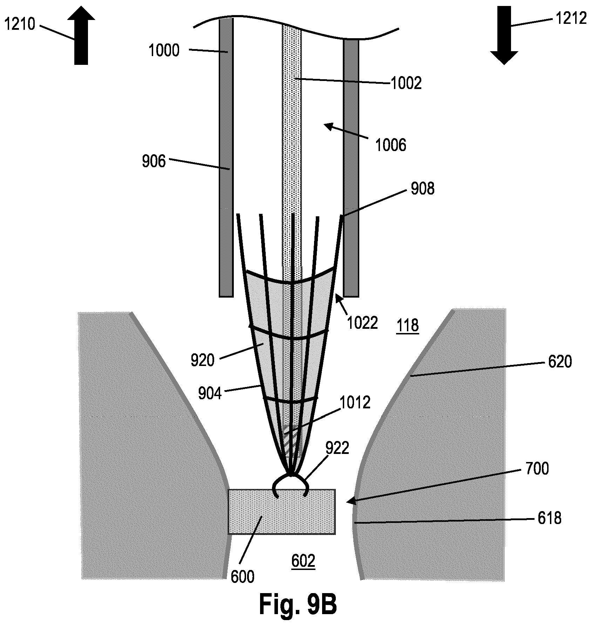

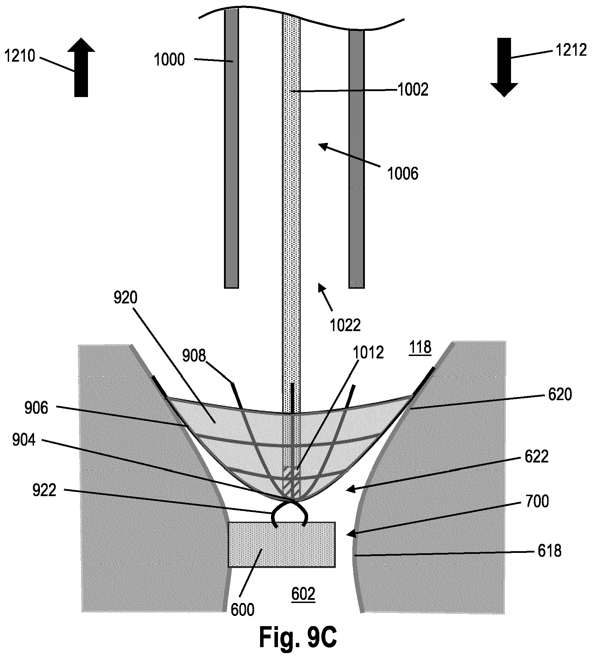

Step 854 of the method 850 may include moving the implantable device 900 out of the distal opening 1022 of the catheter 1000 and over the occlusion device 600 and gap 700. Figs 9B and 9C illustrate two stages of moving the implantable device 900 out of the distal opening 1022 of the catheter 1000.

Fig. 9B illustrates a diagrammatic view of the actuator 1002 moving the bottom 904 and part of the sides 906 of the implantable device 900 out of the distal opening 1022 of the catheter 1000. In some aspects, the actuator 1002 may be moved distally (as shown by arrow 1212) towards the gap 700 to move or push the bottom 904 of the implantable device 900 out of the distal opening 1022 of the catheter 1000. In some aspects, the catheter 1000 may be moved proximally (as shown by arrow 1210) while the actuator 1002 is kept stationary or moved distally, which causes the implantable device 900 to move outside of the lumen 1006. The actuator 1002 may or may not move out of the distal opening 1022 during this step. In some aspects, the implantable device 900 may be pushed out of the distal opening 1022 of the catheter 1000 such that the bottom 904 is disposed proximate to and/or contacts the occlusion device 600. In some aspects, the fastener 922 may contact the occlusion device 600 during this step. For example, the actuator 1002 can be moved longitudinally (e.g., in directions 1210 and/or 1212) and/or the catheter 1000 can be moved laterally (left and/or right in the plane of the page), which would also move the actuator 1002 and the implantable device 900 (because the actuator 1002 and the implantable device 900, in Fig. 9B, coupled to the motion of the catheter 1000) to bring the bottom 904 and/or fastener 922 proximate to and/or into contact with the occlusion device 600. In other aspects, the bottom 904 and/or fastener 922 is spaced from the occlusion device 600 in Fig. 9B and only becomes disposed proximate to and/or contacts the occlusion device 600 later in the process.

In some aspects, the top 908 of the implantable device 900 may remain disposed within the lumen 1006 of the catheter 1000 such that the top 908 and/or sides 906 of the implantable device 900 remain constrained by the walls of the catheter 1000. Thus, the implantable device 900 may still be disposed in the first, constrained configuration. In some aspects, the part of the implantable device 900 outside of the catheter 1000 may expand slightly.

Fig. 9C illustrates a diagrammatic view of the actuator 1002 moving the top 908 out of the implantable device 900 out of the distal opening 1022 of the catheter 1000. In some aspects, the actuator 1002 may be moved distally (as shown by arrow 1212) towards the gap 700 to move or push the top 908 of the implantable device 900 out of the distal opening 1022 of the catheter 1000. The actuator 1002 may move out of the distal opening 1022 during this step. In some aspects, the bottom 904 and/or fastener 922 become disposed proximate to and/or contact the occlusion device 600 only once the implantable device 900 is fully out of the lumen 1006. For example, the actuator 1002 can be moved longitudinally (e.g., in directions 1210 and/or 1212) and/or laterally (left and/or right in the plane of the page), while the implantable device 900 is fully out of the lumen 1006 to bring the bottom 904 and/or fastener 922 proximate to and/or into contact with the occlusion device 600. In Fig. 9C, motion of the actuator 1002 and/or implantable device 900 can be independent of the catheter 1000 because the implantable device 900 is outside of the lumen 1006.

Thus, during this step, the entire implantable device 900 may be moved out of the distal opening 1022 of the catheter 1000 such that the implantable device 900 becomes unconstrained by the walls of the catheter 1000. For example, because the implantable device 900 was elastically deformed when positioned inside the lumen 1006, the implantable device 900 may be biased to return to its original shape when it is outside of the lumen 1006. Thus, the implantable device 900 may expand. In some aspects, the original shape of the implantable device 900 may have a larger diameter than the distance or diameter between walls 620 of the left atrium 118 surrounding the opening 614 of the LAA 602. Thus, as the implantable device 900 moves towards its original shape, the sides 906 and/or top 908 of the implantable device 900 may contact the walls 620 of the left atrium 118. Thus, the implantable device 900 may move, flex, or expand into a second, less constrained configuration in which the implantable device 900 has a larger diameter than in the first, constrained position, but a smaller diameter than in its original shape. In some aspects, the second configuration may be relatively unconstrained when the diameter of between the walls 620 of the left atrium 118 are approximately the same as the diameter of the original shape.

In the second, less constrained configuration, the structure of the implantable device 900 may be biased against the walls 620 of the left atrium such that the mesh 920 may contact the walls 620 of the left atrium 118 and extend across the diameter between the walls 620. Thus, the implantable device 900 may cover the opening 614 of the LAA 602 such that the LAA 602 and, in some aspects, a portion 622 of the left atrium 118 are blocked from the rest of the left atrium 118. In this way, the implantable device 900 may prevent blood flow past the implantable device 900. In some aspects, sides of the implantable device 900 may be symmetrically expanded into contact with the walls 620 of the left atrium 118. In other aspects, the implantable device 900 may be non-symmetrically expanded into contact the walls 620 of the left atrium 118, e.g., one side of the implantable device 900 may be more or less expanded than the other side of the implantable device 900, depending on and/or the location at which the fastener 922 is coupled to the occlusion device 600 and/or the shape of the left atrium 118.

Therefore, this may serve to cover the occlusion device 600 and the gap 700 between the occlusion device 600 and the wall 618 of the LAA 602. The implantable device 900 may be disposed over the LAA 602 and may not directly engage either the LAA 602 or the gap 700. Instead, the implantable device 900 covers the area around the LAA 602 in the left atrium 118 to prevent blood flow from entering the LAA 602 via the gap 700. However, a portion 622 of the left atrium 118 may be blocked off by the implantable device 900. Blood may thus still flow through the gap 700 between the LAA 602 and this covered portion 622 of the left atrium 118. However, the implantable device 900 will prevent blood from the main part of the left atrium 118 from flowing into the covered portion 622 of the left atrium and blood from flowing from the covered portion 622 into the main part of the left atrium 118. Thus, the implantable device 900 may prevent blood from flowing from the main part of the left atrium 118 into the LAA 602 and from the LAA 602 into the left atrium 118 and the rest of the circulatory system. This prevents blood clots from forming in the LAA 602 and/or any blood clots that form in the LAA 602 to move to other parts of the vasculature, despite the presence of a gap 700 between the occlusion device 600 and the walls 618 of the LAA 602.

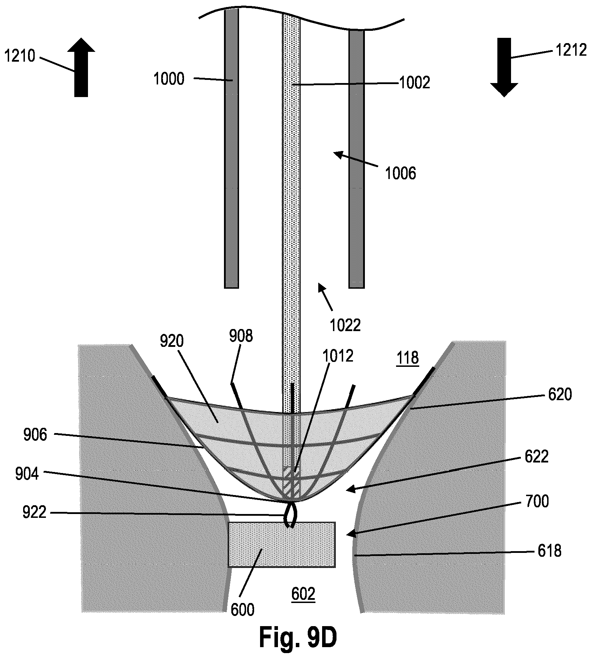

Step 856 of the method 850 may include actuating the fastener 922 to couple the implantable device 900 to the occlusion device 600. Fig. 9D illustrates a diagrammatic view of the actuator 1002 actuating the fastener 922 to couple or attach the implantable device 900 to the occlusion device 600. The fastener 922 may engage, secure, and/or attach to the occlusion device 600 to stabilize the implantable device 900 and hold it in place over the opening 614 of the LAA 602. As described in more detail above, the tip 1012 of the actuator 1002 may actuate the fastener 922 in any suitable way. For example, in the illustrated example, the fastener 922 includes two hooks. Thus, the tip 1012 may be configured to move, bend, or curve the hooks towards each other to capture a portion of the top 608 and/or sides 610 of the occlusion device 600 therebetween. In some aspects, the fastener 922 may couple to the mesh 606 and/or structure 604 of the occlusion device 600. In some aspects, the fastener 922 of the implantable device 900 can contact the occlusion device 600 while the bottom 904 is spaced from the occlusion device 600. In some aspects, both the fastener 922 and the bottom 904 of the implantable device 900 can contact the occlusion device 600. For example, the fastener 922 can extend further into the occlusion device 600.

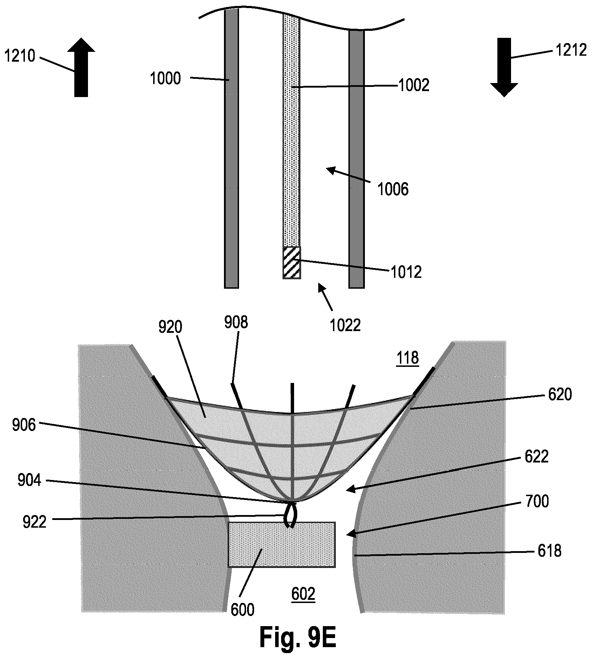

Step 858 of the method 850 may include disengaging the actuator 1002 and/or catheter 1000 from the implantable device 900. Fig. 9E illustrates a diagrammatic view of the actuator 1002 and catheter 1000 disengaging from the implantable device 900. In some aspects, the tip 1012 of the actuator 1002 may disengage, uncouple or move away from the implantable device 900. The catheter 1000 and actuator 1002 may then be moved away from the implantable device 900 (as shown by arrow 1210). In that regard, the implantable device 900 treats, remedies, and/or addresses the undesired blood flow through the gap 700 without the implantable device 900 actually being positioned in the gap (e.g., to fill the space of the gap 700). That is, even with the implantable device 900 is fully deployed, the gap 700 still remains (e.g., the gap 700 is not plugged). This is because the implantable device 900 covers the gap 700 at a location that is locationally spaced from (e.g., proximal of) the longitudinal location of the gap 700).

In some aspects, the actuator 1002 may be moved proximally and may be removed from the catheter 1000 so that a new implantable device 900 can be engaged with the actuator 1002 and inserted into the catheter 1000. Thus, another implantable device 900 can be delivered to and deployed at another gap. Once all of the desired implantable devices 900 have been deployed, the catheter 1000 and actuator 1002 may be removed from the patient. In other aspects, the catheter 1000 and actuator 1002 may be removed completely and a new catheter 1000, actuator 1002, and implantable device 900 may be inserted into the patient to deliver subsequent implantable device 900.

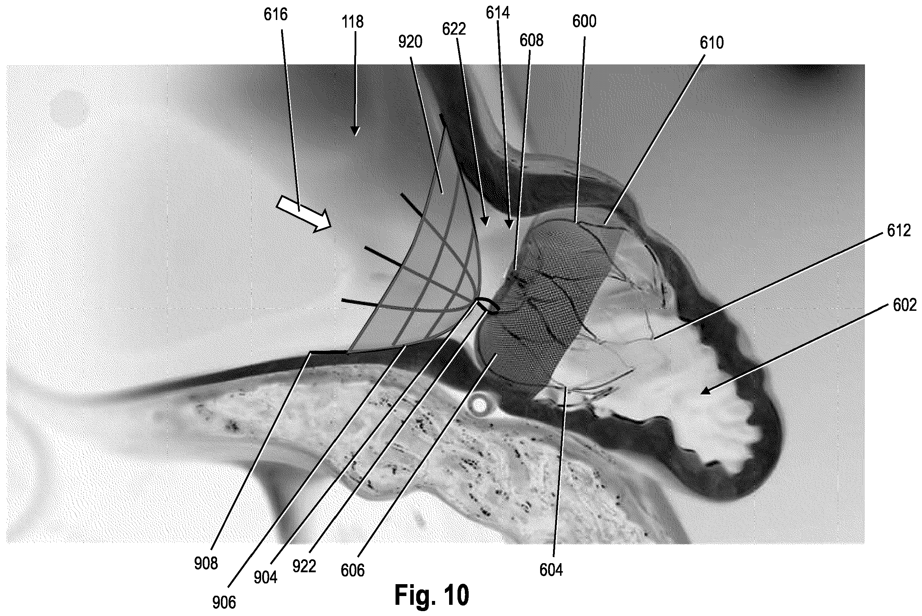

Fig. 10 is a diagrammatic cross-sectional side view of the implantable device 900 and the occlusion device 600 implanted into the left atrium 118 and LAA 602, respectively, according to aspects of the present disclosure. The implantable device 900 is disposed above the opening 614 of the LAA 602. For example, the implantable device 900 is positioned at a location that is locationally spaced (e.g., from the opening 614 of the LAA 602 and/or the occlusion device 600. The sides 906 of the implantable device 900 may contact the walls 620 of the left atrium 118 such that the mesh 920 contacts the walls 620 to cover the LAA 602, the occlusion device 600 implanted therein, and the gap (not shown) disposed therebetween. Thus, a covered portion 622 of the left atrium 118 is disposed between the implantable device 900 and the LAA 602. The implantable device 900 prevents blood flow (as shown by arrow 616) from the left atrium 118 into the covered portion 622 of the left atrium 118, through the gap 700, and into the LAA 602 (and out of the LAA 602, into the left atrium and the rest of the patient's vasculature, in the direction opposite arrow 616). In some aspects, the implantable device 900 is positioned proximate to the LAA 602 and/or the opening 614 of the LAA 602. In some aspects, the implantable device 900 can be positioned completely within the left atrium 118. In some aspects, the implantable device 900 can be positioned partially (e.g., top portion 908) within the left atrium 118 and partially within the LAA 602 and/or the opening 614 of the LAA 602.

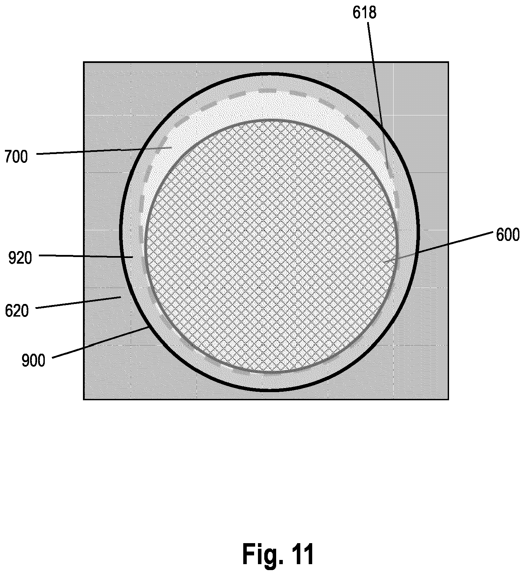

Fig. 11 illustrates a diagrammatic top view of the implantable device 900 and the occlusion device 600 implanted into the left atrium 118 and LAA 602, respectively, according to aspects of the present disclosure. The view in Fig. 10 is similar to the view shown in Fig. 3C, which is taken along section line 3-3 in Fig. 2. In Fig. 10, the wires 902 of the implantable device 900 are not shown for simplicity. The mesh 920 of the implantable device 900 covers both the occlusion device 600 and the gap 700 between the occlusion device 600 and the walls 618 of the LAA 602 to prevent blood flow into the LAA 602 from the main portion of the left atrium. Thus, the implantable device 900 prevents blood flow into the LAA 602 regardless of the shape of the gap 700.

As explained above in reference to Fig. 4, the implantable device 900, catheter 1000, and actuator 1002 together may comprise a leakage treatment subsystem 840 that may be monitored or controlled by a system 800.

The leakage treatment subsystem 840 may include one or more wires that extend through the catheter 1000 and mechanically or electrically couple to one or more of the catheter 1000 or the actuator 1002. In some aspects, the catheter 1000 and/or the actuator 1002 may include one or more sensors and one or more wires may be mechanically or electrically coupled to the one or more sensors. The one or more wires may deliver electrical signals to and/or receive electrical signals from the catheter 1000, actuator 1002, and/or sensors thereof. In some aspects, the leakage treatment subsystem 840 may further include a handle disposed or coupled to the proximal end of the catheter 1000 and/or actuator 1002. The handle may be configured to allow a physician or user to hold and maneuver the leakage treatment subsystem 840. The handle may include one or more buttons or dials or a touchscreen. The handle may be mechanically or electrically coupled to the one or more wires so that the handle is in electrical communication with the catheter 1000, actuator 1002, and/or sensors thereof. The handle may be configured to control movement of the catheter 1000 and or actuator 1002. In some aspects, the handle may be coupled to the proximal end of one or more pullwires and the one or more pullwires may be coupled to a distal portion of the catheter 1000. Thus, the handle may be configured to move the catheter 1000 via the one or more pullwires. The handle may also be coupled to the actuator 1002 such that it controls movement of the actuator 1002 distally/proximally through the catheter 1000 to move the implantable device 900 out of the distal opening 1022 of the catheter 1000. The handle may also be coupled to the actuator 1002 such that it controls actuation or movement of the tip 1012 of the actuator 1002 to actuate the fastener 922 of the implantable device 900 to couple the implantable device 900 to a therapeutic implant 890 (e.g. occlusion device 600). The handle may control the actuator 1002 mechanically or electrically.

The system 800 may include a processing system 810 in communication with the leakage treatment subsystem 840. In some aspects, the processing system 810 is coupled to the leakage treatment subsystem 840 via a wired connection. For example, the processing system 810 may be coupled to the handle of the leakage treatment subsystem 840 via a wire. In other embodiments, the processing system 810 is coupled to the leakage treatment subsystem 840 via a wireless connection including, for example, Wi-Fi or Bluetooth. For example, the handle may include a wireless communication module that receives and/or transmits signals to a wireless communication module of the processing system 810. The processing system 810 may be coupled to an imaging device 860 via a wired or wireless connection. The imaging device 860 may be configured to collect imaging data that shows the catheter 1000, actuator 1002, and/or implantable device 900 to monitor deployment and/or implantation of the implantable device 900. The processing system 810 may be coupled to a display 820 via a wired or wireless connection. The display 820 may display the imaging data so that a user can visualize the catheter 1000, actuator 1002, and/or implantable device 900 within the patient's body (e.g. within the patient's heart). The processing system 810 may also be coupled to an input device 830 via a wired or wireless connection. As explained above in reference to Fig. 4, the input device 830 may include a keyboard, mouse, joystick, microphone, and/or other controller or input device. The user may use the input device 830 to control the display. In some aspects, the user may use the input device to control movement and/or actuation of the catheter 1000, actuator 1002, and/or tip 1012 of the actuator 1002.

In some aspects, the processing system 810 may be configured to send electrical signals and/or data signals to the leakage treatment subsystem 840 to control at least one of movement of the catheter 1000, movement of the actuator 1002 within the catheter 1000, control actuation of the tip 1012 of the actuator 1002, or control the collection of measurements from one or more sensors. The electrical signals and/or data signals may be carried by the wires and/or communicated wirelessly to the leakage treatment subsystem 840. For example, the processing system 810 may transmit a signal in response to a user input via the input device 830. In some aspects, the processing system 810 may be configured to receive signals from the leakage treatment subsystem 840 regarding movement and/or actuation of the catheter 1000, actuator 1002, and/or tip 1012 of the actuator. In some aspects, the processing system 810 may be configured to receive signals and/or measurement data from the one or more sensors. The processing system 810 may use the one or more received signals and/or measurement data to suggest future treatment protocols for the patient (e.g. recommendations for the deployment and/or implantation of the implantable device 900) or predict patient outcomes based on the deployment and/or implantation of the implantable device 900.

Fig. 12 is a schematic diagram of a processor circuit 1600, according to aspects of the present disclosure. The processor circuit 1600 may be implemented in the processing system 810, the system 800, or other devices or workstations (e.g., third-party workstations, network routers, etc.), or on a cloud processor or other remote processing unit, as necessary to implement the method. As shown, the processor circuit 1600 may include a processor 1610, a memory 1612, and a communication module 1614. These elements may be in direct or indirect communication with each other, for example via one or more buses.

The processor 1610 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1610 may also comprise another hardware device, a firmware device, or any combination thereof configured to perform the operations described herein. The processor 1610 may also be implemented as a combination of computing devices, e.g., a combination of a DSP and a microprocessor, a plurality of microprocessors, one or more microprocessors in conjunction with a DSP core, or any other such configuration.

The memory 1612 may include a cache memory (e.g., a cache memory of the processor 1610), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 1612 includes a non-transitory computer-readable medium. The memory 1612 may store instructions 1616. The instructions 1616 may include instructions that, when executed by the processor 1610, cause the processor 1610 to perform the operations described herein. Instructions 1616 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1614 can include any electronic circuitry and/or logic circuitry to facilitate direct or indirect communication of data between the processor circuit 1600, and other processors or devices. In that regard, the communication module 1614 can be an input/output (I/O) device. In some instances, the communication module 1614 facilitates direct or indirect communication between various elements of the processor circuit 1600 and/or the system 800. The communication module 1614 may communicate within the processor circuit 1600 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the leaflet puncture and slitting device) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles), 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

As will be readily appreciated by those having ordinary skill in the art after becoming familiar with the teachings herein, the implantable device 900 advantageously permits a clinician (e.g., a heart surgeon) to reversibly repair leaks that may form during a therapeutic implant 890 (e.g. a replacement valve 300 or an occlusion device 600). Although primarily intended for the repair of leakage of therapeutic implants 890 for the heart, the same technology could be applied to any implant or device where leakage occurs.

The logical operations making up the aspects of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of aspects of the present disclosure. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the present disclosure, e.g., as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. An apparatus, comprising:
an implantable device configured to:
contact heart tissue proximate to a left atrial appendage (LAA) with an occlusion device;
cover a non-circular gap between the occlusion device and a wall of the LAA, and
prevent blood flow into or out of the LAA,
wherein the implantable device comprises:
a frame forming an umbrella-like shape comprising a bottom and sides curving upwards from the bottom;
a mesh disposed over the bottom and at least a portion of the sides of the frame; and
a fastener extending downward from the bottom of the frame and configured to attach to the occlusion device.

2. The apparatus of claim 1, wherein the frame comprises a plurality of wires.

3. The apparatus of claim 1, wherein the frame is collapsable such that:
in a first configuration, the frame comprises a first diameter, and
in a second configuration, the frame comprises a second diameter that is larger than the first diameter.

4. The apparatus of claim 3, wherein the mesh is collapsable with the frame between the second configuration and the first configuration.

5. The apparatus of claim 4, further comprising a delivery catheter comprising a lumen configured to receive the implantable device before the implantable device contacts the tissue.

6. The apparatus of claim 5, further comprising an actuator positioned within the lumen and configured to provide distal motion to the implantable device to move the implantable device outside of the lumen.

7. The apparatus of claim 6, wherein:
when the implantable device is positioned within the lumen, the frame is in the first configuration; and
when the implantable device is moved outside the lumen, the frame is in the second configuration.

8. The apparatus of claim 1, wherein the fastener is one of a suture or a hook comprising at least one bendable wire.

9. The apparatus of claim 1, wherein the mesh is disposed on a convex outer portion of the frame.

10. The apparatus of claim 9, wherein the mesh is disposed over an entirety of the convex outer portion of the frame.

11. A system for treating leakage between a therapeutic device and heart tissue of a patient, comprising:
a catheter comprising a tube defining a lumen and a distal opening at a distal end of the tube; and
the apparatus according to any of the preceding claims.

12. The system of claim 11, further comprising an actuator disposed within the lumen of the catheter and configured to move the implantable device distally within the lumen such that the implantable device exits the catheter via the distal opening.

13. The system of claim 11, wherein the implantable device comprises:
a first, constrained configuration when the implantable device is disposed within the lumen of the catheter; and
a second, unconstrained configuration when the implantable device exits the distal end of the catheter.

14. The system of claim 13, wherein the plurality of wires of the frame are closer to each other in the first configuration than in the second configuration.

15. The system of claim 13, wherein the mesh is flexible such that it is moveable with the frame between the first configuration and the second configuration.
